# EUROPEAN PATENT SPECIFICATION

(11) **EP 3 811 936 B1**
(45) Date of publication and mention of the grant of the patent: **25.06.2025**
(21) Application number: 20189704.8
(22) Date of filing: 05.08.2020
(51) Int. Cl.: A61K 31/137, A61K 31/138, A61K 31/573, A61K 31/711, A61K 31/714, A61K 35/30, A61K 45/06, A61K 9/00, A61P 27/00, A61P 27/02, A61P 27/06

(54) **PARA-BULBAR PREPARATION WITH CYTOTROPHIC-RETINAL EFFECT**
PARA-BULBAR PRÄPARAT MIT ZYTOTROPHE-RETINALE WIRKUNG
PRÈPARATION PARA-BULBAIRE À EFFET CYTOTROPHIQUE-RÉTINIEN

(30) Priority: 05.08.2019 IT 201900014052
(43) Date of publication of application: 28.04.2021
(73) Proprietor: Scorolli, Lucia, 40141 Bologna (BO) (IT)
(72) Inventor: Scorolli, Lucia, 40141 Bologna (BO) (IT)
(74) Representative: Ruzzu, Giammario

(56) References cited:
- EP-A1- 1 380 302
- EP-B1- 1 380 302
- WO-A2-2007/060458
- WO-A2-2007/150046
- RU-C1- 2 495 669
- EMINE SEDA GUVENDAG GUVEN ET AL: "Angle closure glaucoma induced by ritodrine", ACTA OBSTETRICIA AND GYNECOLOGICA SCANDINAVICA., vol. 84, no. 5, 12 April 2005 (2005-04-12), DK, pages 489 - 490, XP055688187, ISSN: 0001-6349, DOI: 10.1111/j.0001-6349.2005.0243a.x

## Description

### DESCRIPTION OF THE INVENTION

The present invention relates to a preparation for para-bulbar administration with a cytotrophic-retinal (or cytotropic-retinal) effect.

In particular, the present invention refers to a mixture of already existing active principles of clinical efficacy verified in the treatment both of glaucomatous retino-opticopathies, both in case of atrophic macular degeneration, and in case of neovascular maculopathy after vascular treatment.

### PRIOR ART RELATING TO THE INVENTION

For many years, the search for new products in the field of glaucomatous retino-optic disease treatment required an enormous scientific, economic and industrial effort.

In fact, in order to obtain results of ever greater efficacy and rapidity of effects, the research required the identification of active ingredients and administration techniques, as well as the conduct of subsequent biological tests and "field" and clinical application trials.

Glaucomatous retino-opticopathies are often due to an increase in endobulbar pressure resulting from a defective aqueous outflow.

In the normal eye there is a flow of aqueous humor which, through an active transport mechanism, is conveyed through the normal endothelium and entered into the lumen of Schlemm's canal. From here, the aqueous is drained through small openings in the outer wall to the scleral collectors and into the capillaries of the subconjunctival tissues.

In the eye affected by glaucoma, the endothelium of Schlemm's canal is more resistant and so does the adjacent trabecular tissue.

For this reason, the passage of water becomes very slow, which leads to an increase in intraocular pressure.

This involves suffering, and can lead to atrophy of the optic nerve fibers and optic ganglion cells, which give them origin.

Under normal conditions, the number of ganglion cells decreases physiologically and progressively during the life of each individual, without however causing characteristic functional deficits.

The physiological loss of ganglion cells does not lead to damage of the visual field, since there is a certain reserve and overlapping of cell fields, which carry the stimuli of the same retinal area.

In glaucomatous disease, the physiological loss due to the disease is added to the physiological loss, causing the subject to lose many ganglion cells so that the reserve is no longer sufficient to guarantee the integrity of the visual function, thus leading to the formation of perimeter deficits.

The loss of ganglion cells therefore leads to a slow and progressive atrophy of the optic disc.

Atrophic macular degeneration is a pathological condition related to progressive apoptosis with progressive rarefaction of the macular retina, due both to an imbalance of the phagocytic capacity of the pigment epithelium and to a mitochondrial enzymatic reduction, of which, however, the genetics are not yet known .

It is a disease that affects the macula, or the central area of the retina, and almost always affects both eyes.

The disease presents with a progressive course that can lead to irreversible loss of central vision.

Yellowish lesions of the retina, detectable during the examination of the ocular fundus, are the characteristic precursor signs of the disease with the formation of drusen.

The most common correlated phenomenon is the aging process of the eye: the macula, containing numerous photoreceptors (cones are concentrated there), alters until it loses its characteristics.

This is due to retinal cell death, which can be slow and progressive or more rapid and dramatic.

To get an idea of the importance of this pathology, just think that in industrialized countries it represents the first cause of low vision in people over the age of 50.

It is expected that in the next few years there will be hundreds of millions of people affected by atrophic macular degeneration, a figure that is likely to grow with the world's aging population (especially in the most prosperous countries).

Edematous (neovascular) maculopathy is also a pathology that attacks the area of the macula.

It is due to a metabolic suffering of the pigment epithelium, of the retinal and macular cells, and causes a neovascular push with the formation of tokens of newly formed capillaries that, from the choroid, progressively invade the subretinal spaces and grow inside the retina.

Therefore, since we are dealing with newly formed capillaries, an oozing in these spaces ensues, and the liquid that comes out of these abnormal vessels accumulates in the central retina (the macula in fact), causing distortion of the images.

In fact, precisely because of the macular edema that arises following the leakage of fluid from the blood vessels of the macula, the macula itself swells and / or thickens, favouring the appearance of vision problems.

The treatments used up to now to contrast these pathological situations foresee in most cases endobulbar injections of active ingredients, such as vascular antiproliferatives.

The endobulbar injections, however, involve surgical risks of considerable importance, without however excluding possible relapses.

RU 2 495 669 C1 discloses the para-bulbar administration of Coenzyme Compositum^{®} for the treatment of degenerative and dystrophic retinal diseases including TSHRD, PHRD, AMD (dry form), optic atrophy, glaucoma, retinitis pigmentosa and diabetic angioretinopathy.

EP-A-1 380 302 discloses the use of betamethasone for the treatment of macular degeneration.

WO 2007/060458 discloses the use of ritodrine for the treatment of macular degeneration.

WO 2007/150046 discloses the use of compositions comprising phospholipids in the treatment of macular degeneration.

Acta Obstet. Gynecol. Scand. 2005, 84(5), 489 discloses the induction of glaucoma in a patient from the use of ritodrine.

### OBJECTS OF THE INVENTION

The object of the present invention is to propose a preparation which can be used for the treatment of the aforementioned pathologies with verified efficacy, which can be applied safely and which is able to lengthen the time of the risk of relapse.

Finally, an aim of the present invention is to propose a package containing the aforementioned mixture, which allows easy and effective administration, reducing surgical risks.

### SUMMARY OF THE INVENTION

These and other purposes are fully achieved by means of a para-bulbar preparation with a cytotrophic-retinal effect, consisting of a mixture of the following active ingredients:
a) betamethasone;
b) isoxsuprine hydrochloride;
c) phospholipids isolated from the cerebral cortex and cyanocobalamin;
d) polydeoxyribonucleotide.

The invention is defined by the claims.

### BRIEF DESCRIPTION OF THE DRAWINGS

The invention will be described below in detail, in accordance with the content of the claims and, as regards examples of clinical cases in which the invention has been applied for experimental purposes, with the aid of the attached drawing tables, in which:
- Figures 1A and 1B concern the results of as many visual field analyzes, carried out respectively before and after the application of the para-bulbar preparation of the invention, in a first formulation;
- Figures 2A and 2B concern the results of as many OCT examinations, carried out respectively before and after the application of the para-bulbar preparation of the invention, in a second formulation thereof..

### DETAILED DESCRIPTION OF PREFERRED EMBODIMENTS OF THE INVENTION

In accordance with the present invention, a para-bulbar preparation with a cytotrophic-retinal effect is provided consisting of a mixture of the following active ingredients:
a) betamethasone;
b) isoxysuprine hydrochloride
c) phospholipids isolated from the cerebral cortex and cyanocobalamin;
d) polydeoxyribonucleotide.

Betamethasone, better known on the market under the commercial name of Bentelan^{®}, acts by neutralizing the pro-inflammatory effects deriving from cellular suffering and the consequent accumulation of metabolic waste.

The quantity of betamethasone foreseen in the mixture object of the invention is 4 mg equal to 2 ml (s.i.) (5 ml in the 50 ml syringe, 30 G needle). Isoxsuprine hydrochloride (beta-sympathetic-mimetic), is a vasodilator, and has the function of promoting the extent of perfusion of the remaining drugs in the retino-papillary endobulbar districts.

The quantity of isoxsuprine hydrochloride expected in the mixture is 20 ml (s.i.).

Cerebral cortex phospholipids supplemented with cyanocobalamin produce a supporting effect on cell membranes and stabilizing DNA.

The compound has often been used off-label. Off-label means the use in clinical practice of drugs outside the conditions authorized by the bodies set up for pathology, population or dosage.

Generally off-label use concerns molecules already known and used for some time, for which there is considerable scientific evidence that would allow their rational use even in clinical situations in ways not provided for in the relative technical data sheet.

Off-label prescribing is also achieved when a drug is administered differently from the authorized administration methods, both in terms of time and quantity.

The expected amount of phospholipids in the mixture is 12 mg while the amount of cyanocobalamin is 1 mg, equal to 2 ml (s.i.). The total to be injected is 10 ml.

In a first formulation of the para-bulbar preparation, the introduction in the above mentioned mixture of polydeoxyribonucleotide is foreseen, which determines a positive effect on the cell trophism by means of the PDRN metabolites on the activation of the anti-atrophic and trophic cell growth factors.

The quantity of polydeoxyribonucleotide foreseen in the mixture object of the present invention amounts to 5.625 mg, equal to 3 ml (s.i.). The total to be injected is 15 ml.

Overall, the total to be injected of the compound is 0.5 ml equal to 50 U.

In accordance with the present invention, a package is also provided containing the active ingredients of the aforementioned mixture, already dosed and prepared for administration.

The proposed package is in the single-dose version, and can consist of a sterile syringe, with a piston locking device.

According to an advantageous variant, the piston locking device is made of plastic material, thus reducing production costs.

In practice, it is a matter of collecting the four active ingredients that make up the mixture object of the invention in a single sterile vial or syringe, already prepared for carrying out the injection of the mixture itself.

The parabulbar injection should be performed in the infero-temporal sector, after inviting the patient to look in the opposite direction.

In this location the fibrous septae are less numerous and oriented longitudinally, thus favouring the perfusion of the liquid towards the posterior pole of the eye.

Here the liquid meets the crown of posterior ciliary vessels entering the bulb, thus favoring the penetration of the mixture into the endobulbar region.

This type of injection avoids the parabulbar stagnation of the injecting liquid, which would hinder the absorption of the latter.

Another reason that justifies the choice of making the substances of the mixture injective into the orbit is that this allows direct absorption, and therefore a much higher opportunity than that which would occur in general, having in the latter way to overcome the blood-brain barrier.

Studies carried out by the inventors on numerous patients treated over a period of several years during his clinical experience, lead us to believe that the intended purposes have been fully achieved.

The optimal dosage, after application on over 15,000 patients, proved to be that of one infiltration per week for six consecutive weeks, at the end of which the first effects are seen with visual acuity, OCT and visual field. Sometimes, in particularly weakened eyes, the first cycle consists of 10 injections.

However, the objective OCT data was considered as the basal parameter for the evaluation, given that the other two parameters can be easily influenced due to psychosensory effects.

Two clinical cases are described below, by way of example, the results of which are particularly significant in demonstrating the positive effects of the mixture object of the invention.

Example 1 relates to a case of application of the parabulbar preparation carried out using the above described first formulation of the mixture; Example 2 relates to a case of application of the parabulbar preparation carried out using the above described second formulation of the mixture.

### EXAMPLE 1

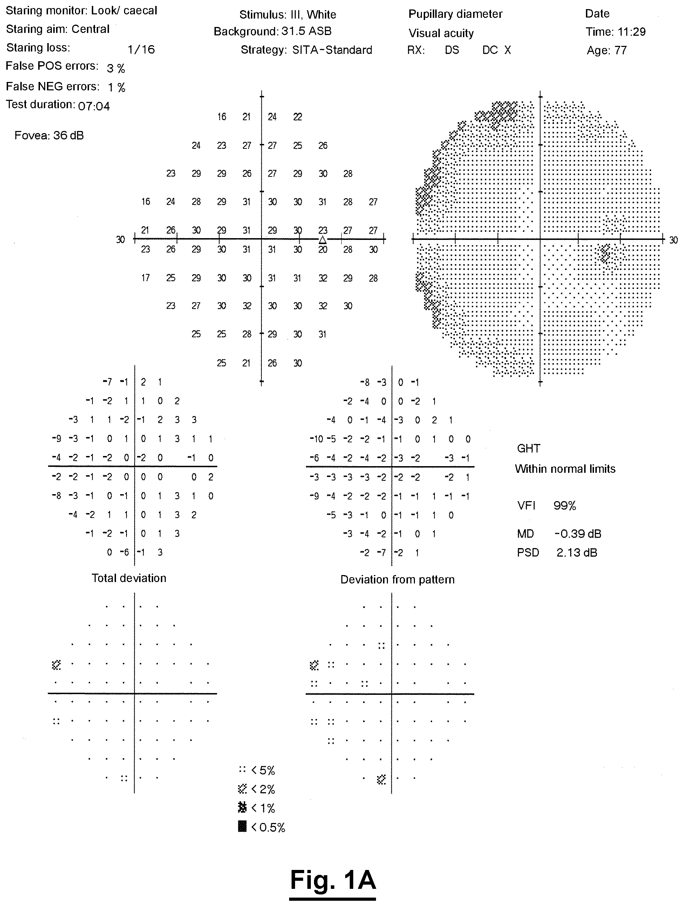

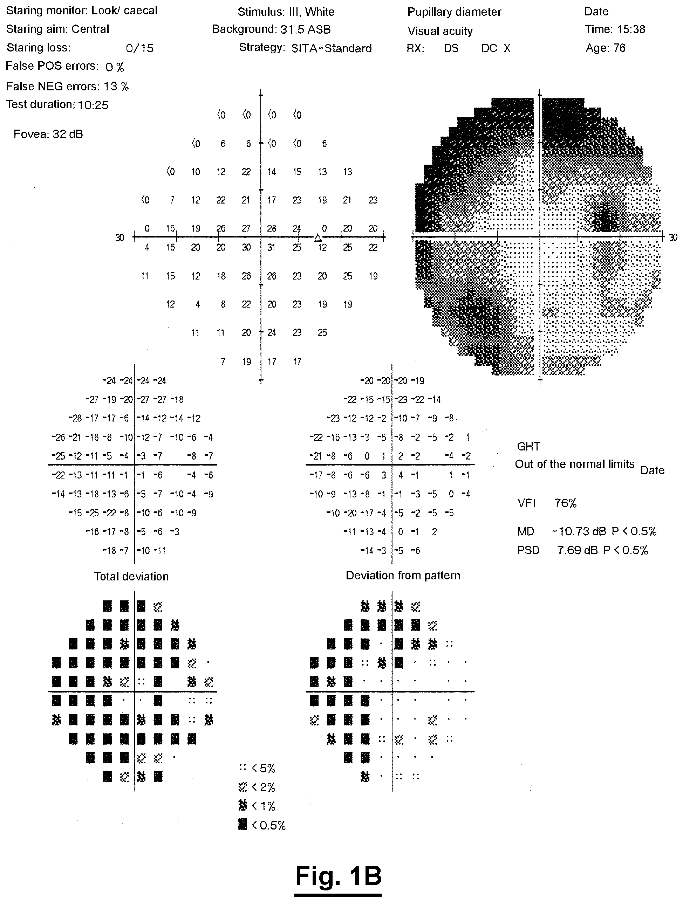

A clinical case of chronic right glaucoma was treated with the mixed para-bulbar preparation according to the first formulation of the invention. The pre- and post-treatment situation is illustrated, respectively, by visual field studies carried out approximately 9 months apart, the results of which are shown in Figures 1A and 1B.

### 76 year old female subject.

Diagnosis: chronic simple glaucoma of the right eye.

Situation prior to treatment (see figure 1A): the patient presented an objective worsening of the clinical picture over time, despite having been subjected to conventional therapy, and the ocular pressure had been kept within acceptable values. The study of the visual field shows values well outside the normal limits, essentially on all quadrants.

Treatment: administered 3 cycles of injections, 4 months apart; in the first cycle 10 injections of the mixture were administered, in the second and third cycles 6 injections were administered.

Mixture injected for each:
a) betamethasone: 4 mg / 2 ml (s.i.) = 0.05 ml ;
b) isoxsuprine hydrochloride: 2 ml (s.i.) = 0.20 ml;
c) phospholipids from the cerebral cortex added to cyanocobalamin: 12 mg e 1 mg/2 ml respectively, (s.i.) = 10 U = 0.10 ml;
d) polydeoxyribonucleotide: 5.625 mg/3 ml (s.i.) = 15 U = 0.15 ml.

Post-treatment situation: (see figure 1B): there is a notable improvement in sharpness and contrast, with clear recovery of perimeter sensitivity. Basically, the values in all quadrants of the visual field turn out to be within normal limits.

### EXAMPLE 2 (Comparative Example)

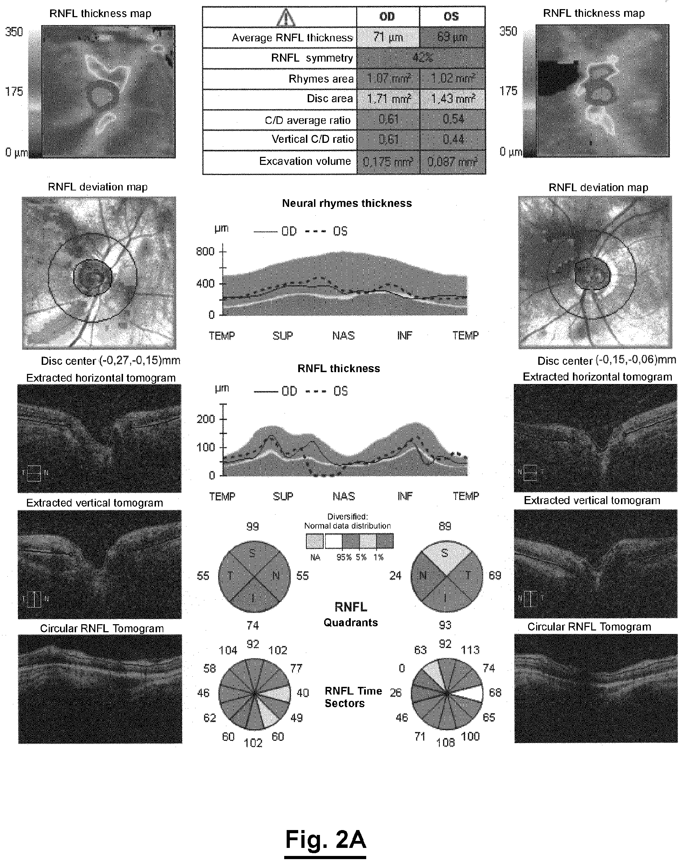

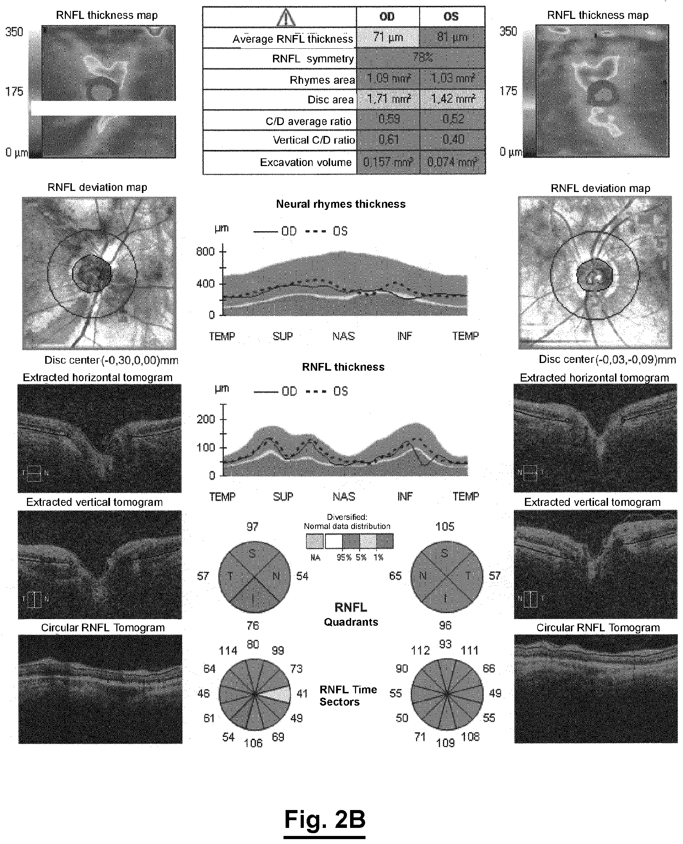

A clinical case of chronic left glaucoma. was treated with the mixed parabulbar preparation as described for the second formulation of the invention. The pre- and post-treatment situation is illustrated, respectively, by OCT examinations carried out approximately 9 months apart, the results of which are shown in Figures 2A and 2B.

### 61-year-old male.

### Diagnosis: chronic simple glaucoma of the left eye.

Situation prior to treatment (see figure 2A): the patient presented an objective worsening of the clinical picture over time, despite having been subjected to conventional therapy, and the ocular pressure had been kept within acceptable values. The OCT examination shows, for what concerns the left eye, reduced fiber thickness values, in some sectors with losses significantly greater than 30%.

Treatment: administered 3 cycles of injections, 4 months apart; 6 injections were administered in each cycle.

Mixture injected for each:
a) betamethasone: 4 mg/2 ml (s.i.) = 0.05 ml;
b) isoxsuprine hydrochloride: 2 ml (s.i.) = 0.20 ml;
c) phospholipids from the cerebral cortex added to cyanocobalamin: 12 mg e 1 mg/2 ml respectively, (s.i.) = 10 U = 0.10 ml;
d) Coenzyme Compositum^{®} 0.15 ml.

Post-treatment situation: (see figure 2B): there is a total recovery of the thickness of the fibers, with values of the left eye substantially comparable with those measured for the right eye, and in some hourly sectors RNFL even higher.

It should be noted that in the forms of neuro vascular maculopathies the treatment described and illustrated drastically reduces relapses, thus reducing the costs and the intrinsic surgical risk of endobulbar injection..

## Claims

1. A preparation for para-bulbar administration, for use in the treatment of glaucomatous retino-opticopathies, both of atrophic macular degeneration and in case of neovascular maculopathy after vascular treatment, **characterized in that** it consists of a mixture of the following active ingredients:
a) betamethasone;
b) isoxsuprine hydrochloride;
c) a mixture or an adduct of phospholipids isolated from the cerebral cortex and cyanocobalamin;
d) polydeoxyribonucleotide.

2. A preparation for use according to claim 1, wherein the various components of said mixture are provided according to the following quantities:
a) betamethasone: 4 mg / 2 ml = 0.05 ml injectable;
b) isoxsuprine hydrochloride: 2 ml = 0.20 ml injectable;
c) a mixture or an adduct of phospholipids isolated from the cerebral cortex and cyanocobalamin: 12 mg and 1 mg / 2 ml respectively = 10 U = 0.10 ml injectable;
d) polydeoxyribonucleotide: 5.625 mg / 3 ml = 15 U = 0.15 ml injectable.

3. Package containing the active ingredients of one or more of the preceding claims, designed to contain a single dose.

4. Package according to claim 3, of the single-dose type, consisting of a sterile syringe or vial with a piston locking device.

5. Package according to claim 4, in which a piston locking device made of plastic material is provided.

## Patentansprüche

1. Präparat zur para-bulbären Verabreichung, zur Verwendung bei der Behandlung von glaukomatösen Retino-Optikopathien, sowohl bei atrophischer Makuladegeneration als auch bei neovaskulärer Makulopathie nach vaskulärer Behandlung,
**dadurch gekennzeichnet, dass** es aus einer Mischung der folgenden Wirkstoffe besteht:
a) Betamethason;
b) Isoxsuprin-Hydrochlorid;
c) einer Mischung oder einem Addukt aus Phospholipiden, die aus der Großhirnrinde isoliert wurden, und Cyanocobalamin;
d) Polydeoxyribonukleotid.

2. Präparat zur Verwendung nach Anspruch 1, in der die verschiedenen Bestandteile der Mischung in den folgenden Mengen bereitgestellt werden:
a) Betamethason: 4 mg / 2 ml = 0,05 ml injizierbar;
b) Isoxsuprinhydrochlorid: 2 ml = 0,20 ml injizierbar;
c) eine Mischung oder ein Addukt von aus der Großhirnrinde isolierten Phospholipiden und Cyanocobalamin:
12 mg bzw. 1 mg / 2 ml = 10 U = 0,10 ml injizierbar;
d) Polydeoxyribonukleotid: 5,625 mg / 3 ml = 15 U = 0,15 ml injizierbar.

3. Packung, die die Wirkstoffe eines oder mehrerer der vorhergehenden Ansprüche enthält und zur Aufnahme einer Einzeldosis bestimmt ist.

4. Packung nach Anspruch 3, vom Einzeldosis-Typ, bestehend aus einer sterilen Spritze oder Durchstechflasche mit einer Kolbenverriegelungsvorrichtung.

5. Packung nach Anspruch 4, in der eine Kolbenverriegelungsvorrichtung aus Kunststoff vorgesehen ist.

## Revendications

1. Préparation pour administration para-bulbaire, destinée au traitement des rétino-opticopathies glaucomateuses, de la dégénérescence maculaire atrophique et de la maculopathie néovasculaire après traitement vasculaire, **caractérisé par le fait qu'**il est constitué d'un mélange des principes actifs suivants:
a) bétaméthasone;
b) chlorhydrate d'isoxsuprine;
c) un mélange ou un adduit de phospholipides isolés du cortex cérébral et de cyanocobalamine;
d) polydésoxyribonucléotide.

2. Préparation utilisable selon la revendication 1, dans laquelle les différents composants dudit mélange sont apportés selon les quantités suivantes:
a) bétaméthasone: 4 mg / 2 ml = 0,05 ml injectable;
b) chlorhydrate d'isoxsuprine: 2 ml = 0,20 ml injectable;
c) un mélange ou un adduit de phospholipides isolés du cortex cérébral et de cyanocobalamine: 12 mg et 1 mg / 2 ml respectivement = 10 U = 0,10 ml injectable;
d) polydésoxyribonucléotide: 5,625 mg / 3 ml = 15 U = 0,15 ml injectable.

3. Emballage contenant les principes actifs d'une ou plusieurs des revendications précédentes, destiné à contenir une dose unique.

4. Emballage selon la revendication 3, de type monodose, constitué d'une seringue ou d'un flacon stérile avec un dispositif de blocage du piston.

5. Emballage selon la revendication 4, dans lequel un dispositif de verrouillage du piston en matière plastique est prévu.
